(19) Europäisches Patentamt

European Patent Office

Office européen des brevets

(11) EP 0 683 662 B1

(12) EUROPÄISCHE PATENTSCHRIFT

(45) Veröffentlichungstag und Bekanntmachung des Hinweises auf die Patenterteilung:
17.06.1998 Patentblatt 1998/25

(21) Anmeldenummer: 94906172.5

(22) Anmeldetag: 29.01.1994

(51) Int. Cl.$^6$: A61K 7/42

(86) Internationale Anmeldenummer:
PCT/EP94/00257

(87) Internationale Veröffentlichungsnummer:
WO 94/17780 (18.08.1994 Gazette 1994/19)

(54) **WASSERFESTE KOSMETISCHE ODER DERMATOLOGISCHE LICHTSCHUTZZUBEREITUNGEN**

WATERPROOF COSMETIC OR DERMATOLOGICAL PHOTOPROTECTIVE PREPARATIONS

FORMULATIONS COSMETIQUES OU DERMATOLOGIQUES DE PROTECTION SOLAIRE RESISTANT A L'EAU

(84) Benannte Vertragsstaaten:
AT BE CH DE DK ES FR GB GR IT LI NL SE

(30) Priorität: 11.02.1993 DE 4303983
15.12.1993 DE 4342719

(43) Veröffentlichungstag der Anmeldung:
29.11.1995 Patentblatt 1995/48

(73) Patentinhaber:
Beiersdorf Aktiengesellschaft
20245 Hamburg (DE)

(72) Erfinder:
• GERS-BARLAG, Heinrich
D-25495 Kummerfeld (DE)
• HACHMANN, Stefan
D-22846 Norderstedt (DE)
• NISSEN, Bente
D-20146 Hamburg (DE)
• SCHULZ, Sabine
D-22529 Hamburg (DE)

(56) Entgegenhaltungen:
WO-A-90/09777          DE-A- 3 824 999
GB-A- 2 206 282        US-A- 4 731 242
US-A- 5 207 998

**Beschreibung**

Die vorliegende Erfindung betrifft wasserfeste kosmetische bzw. dermatologische Lichtschutzzubereitungen, insbesondere solche Lichtschutzzubereitungen in Form von Emulsionen oder Hydrodispersionen.

Unter kosmetischer Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der kosmetischen Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwetteinflüssen, insbesondere vor Sonne und Wind, schützen und die Anzeichen der Hautalterung verzögern.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen der Haut.

Als ein Maximum der Erythemwirksamkeit des Sonnenlichtes wird der engere Bereich um 308 nm angesehen.

Zum Schutze gegen UVB-Strahlung sind zahlreiche Verbindungen bekannt, bei denen es sich zumeist um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Wellenlängenbereich zwischen etwa 320 und 400 nm, den sogenannten UVA-Bereich, sind UV-Filtersubstanzen wichtig, da auch solche Strahlen Schäden hervorrufen können. So ist erwiesen, daß UVA-Strahlung zu einer Schädigung der elastischen und kollagenen Fasern des Bindegewebes führt, was die Haut vorzeitig altern läßt und als Ursache zahlreicher phototoxischer und photoallergischer Reaktionen zu sehen ist. Der schädigende Einfluß der UVB-Strahlung kann durch UVA-Strahlung verstärkt werden.

Die UV-Strahlung kann aber auch zu photochemischen Reaktionen führen, wobei dann die photochemischen Reaktionsprodukte in den Haut- und Zellmetabolismus eingreifen.

Vorwiegend handelt es sich bei solchen photochemischen Reaktionsprodukten um radikalische Verbindungen, z.B. Hydroxyradikale, Hydroperoxyradikale sowie Superoxidionen. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls, kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Um diesen Reaktionen vorzubeugen, können den kosmetischen bzw. dermatologischen Formulierungen zusätzlich Antioxidantien und/oder Radikalfänger einverleibt werden.

UV-Absorber bzw. UV-Reflektoren sind die meisten anorganischen Pigmente, die bekannterweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen.

Obwohl es durchaus vorteilhafte kosmetische bzw. dermatologische Zubereitungen zum Schutze der Haut vor den schädlichen Folgen der Einwirkung von UV-Licht gibt, ist ein oft beobachteter Nachteil, daß die Zubereitungen nicht oder nicht hinreichend wasserfest sind.

Lichtschutzzubereitungen werden besonders häufig an Badestränden bzw. in Freibädern benötigt und angewandt. Wünschenswert ist dann, daß die Lichtschutzformulierung weitgehend wasserfest ist, daß sie also nicht oder nur in geringem Maße von der Haut abgewaschen wird.

Höhere Lichtschutzfaktoren, also etwa solche die oberhalb von LF 15 angesiedelt sind, lassen sich im allgemeinen nur durch hohe Mengen an UV-Filtersubstanzen erreichen. Soll ein Sonnenschutzprodukt auch nach dem Baden noch einen hohen Lichtschutzfaktor aufweisen, muß insbesondere die UV-Filtersubstanz auf der Haut erhalten bleiben.

Es ist an sich schon lästig, wenn nach dem Baden das Sonnenschutzprodukt erneut aufgetragen werden muß. Beim Baden selbst kann die Verwendung einer abwaschbaren Lichtschutzformulierung unter Umständen sogar leichtsinnig und schädlich für die Haut sein, da Wasser das Licht im UVA- und UVB-Bereich schlecht absorbiert, infolge dessen keinen nennenswerten UV-Schutz darstellt, nicht einmal für untergetauchte Hautbereiche.

Für wasserfeste Lichtschutzformulierungen verwendet der Stand der Technik üblicherweise nichtwasserlösliche UV-Filtersubstanzen, wasserabweisende Rohstoffe (z.B. Siliconöle in hohen Konzentrationen) und/oder Filmbildner, insbesondere hochmolekulare Verbindungen (z.B. PVP/Hexadecen-Copolymere). Dabei werden Barrieren zwischen den auf der Haut aufliegenden UV-Filtersubstanzen und dem Wasser aufgebaut.

Nachteilig dabei ist, daß die Diffusion der Filtersubstanzen ins Wasser zwar verzögert, aber nicht vollständig verhindert werden kann. Deshalb können derartige Produkte bei längerem Baden beachtlich an Schutzwirkung verlieren.

Anorganische Pigmente zeichnen sich zwar an sich durch gute Lichtschutzwirkung aus. Sie haben jedoch den Nachteil, daß es im allgemeinen schwierig ist, sie in befriedigender Weise solchen Formulierungen einzuverleiben.

Ein weiterer Nachteil des Einsatzes anorganischer Pigmente in kosmetischen Formulierungen ist, daß solche Pigmente in den weitaus meisten Fällen zu starker Hauttrokkenheit führen.

Da sinnvollerweise verhindert werden muß, daß sich die Pigmentkörner zu Agglomeraten zusammenballen, mußte den Formulierungen stets ein gewisser Anteil an Emulgatoren oder vergleichbaren ober- oder grenzflächenaktiven Substanzen zugefügt werden.

Übliche kosmetische Darreichungsformen sind Emulsionen, also metastabile Zwei- oder Mehrphasensysteme bei welchen die einzelnen Phasen im flüssigen Zustande vorliegen. Ein gängiger Emulsionstyp sind O/W-Emulsionen.

An sich ist die Verwendung der üblichen kosmetischen Emulgatoren unbedenklich. Den-noch können Emulgatoren, wie letztlich jede chemische Substanz, im Einzelfalle allergische oder auf Überempfindlichkeit des Anwenders beruhende Reaktionen hervorrufen.

So ist bekannt, daß bestimmte Lichtdermatosen durch gewisse Emulgatoren, aber auch durch verschiedene Fette, und gleichzeitige Exposition von Sonnenlicht ausgelöst werden. Solche Lichtdermatosen werden auch "Mallorca-Akne"genannt. Eine Aufgabe der vorliegenden Erfindung war daher, wasserfeste Lichtschutzzubereitungen zu entwikkeln, welche auch als emulgatorfreie Präparate Anwendung finden können.

Emulgatorfreie Lichtschutzpräparate auf Basis sogenannter Hydrodispersionen sind seit einiger Zeit für den Verbraucher zugängig.

Hydrodispersionen stellen Dispersionen einer flüssigen, halbfesten oder festen inneren (diskontinuierlichen) Lipidphase in einer äußeren wäßrigen (kontinuierlichen) Phase dar.

Im Gegensatze zu O/W-Emulsionen, die sich durch eine ähnliche Phasenanordnung auszeichnen, sind Hydrodispersionen aber im wesentlichen frei von Emulgatoren. Hydrodispersionen stellen, wie im übrigen auch Emulsionen metastabile Systeme dar, und sind geneigt, in einen Zustand zweier in sich zusammenhängender diskreter Phasen überzugehen. In Emulsionen verhindert die Wahl eines geeigneten Emulgators die Phasentrennung.

Bei Hydrodispersionen einer flüssigen Lipidphase in einer äußeren wäßrigen Phase kann die die Stabilität eines solchen Systems beispielsweise dadurch gewährleistet werden, daß in der wäßrigen Phase ein Gelgerüst aufgebaut wird, in welchem die Lipidtröpfchen stabil suspendiert sind.

Obwohl dieser Formulierungstyp gegenüber den herkömmlichen Lichtschutzformulierungen den Vorteil der Emulgatorfreiheit bietet, gibt es andererseits auch einige Punkte, welche der Verbesserung bedürfen. So ist für eine gute Lichtschutzwirkung solcher Präparate eine vergleichsweise hohe Konzentration an UV-Filtern notwendig. Darüberhinaus fühlen sich solche Präparate im Vergleich zu Emulsionen und Lichtschutzölen meist klebrig an.

Ein klebriges Sonnenschutzprodukt aber ist gerade bei sommerlicher Hitze, also der gewöhnlichen Einsatztemperatur solcher Formulierungen, besonders unangenehm, zumal, wenn auch noch Sandpartikel auf die behandelte Haut geraten.

Eine weitere Aufgabe war daher, wasserfeste Lichtschutzpräparate zur Verfügung zu stellen, welche gewünschtenfalls eine relativ niedrige Konzentration an UV-Filtern aufweisen können und darüberhinaus ein angenehmes Hautgefühl vermitteln. Noch eine weitere Aufgabe der vorliegenden Erfindung bestand darin, nichtklebrige Lichtschutzpräparate zur Verfügung zu stellen.

Es hat sich erstaunlicherweise herausgestellt, und darin liegt die Lösung all dieser Aufgaben begründet, daß wasserfeste kosmetische oder dermatologische Lichtschutzformulierungen in Form von O/W-Emulsionen oder Hydrodispersionen, enthaltend

- ein oder mehrere kosmetisch oder pharmazeutisch unbedenkliche hydrophobe anorganische Pigmente, wobei diese Pigmente in die Ölphase der Emulsionen bzw. Hydrodispersionen eingebaut sind,

  - wobei, falls Titandioxid als hydrophobes anorganisches Pigment eingesetzt wird, die Hydrophobierung des Titandioxids nicht auf einer Aluminiumstearatbehandlung beruht

- eine oder mehrere kosmetisch oder pharmazeutisch unbedenkliche öllösliche UV-Filtersubstanzen,
- einen oder mehrere Filmbildner
  ferner gegebenenfalls enthaltend
- eine oder mehrere kosmetisch oder pharmazeutisch unbedenkliche wasserlösliche UV-Filtersubstanzen

- eine oder mehrere Substanzen, gewählt aus der Gruppe der üblichen kosmetischen oder dermatologischen Wirk- , Hilfs- und/oder Zusatzstoffe
  in einem üblichen kosmetischen oder pharmzeutischen Träger,
  den Mißständen des Standes der Technik abhelfen.

Vorteilhafte Ausführungsformen der vorliegenden Erfindung sind im besonderen wasserfeste Lichtschutzformulierungen in Form von Hydrodispersionen, welche aus einer inneren Lipidphase und einer äußeren wäßrigen Phase bestehen, und welche im wesentlichen frei von Emulgatoren sind, enthaltend

- ein oder mehrere kosmetisch oder pharmazeutisch unbedenkliche hydrophobe anorganische Pigmente, wobei diese Pigmente in die Ölphase der Emulsionen bzw. Hydrodispersionen eingebaut sind,

  - wobei, falls Titandioxid als hydrophobes anorganisches Pigment eingesetzt wird, die Hydrophobierung des Titandioxids nicht auf einer Aluminiumstearatbehandlung beruht

- eine oder mehrere kosmetisch oder pharmazeutisch unbedenkliche öllösliche UV-Filtersubstanzen,
- einen oder mehrere Filmbildner
  ferner gegebenenfalls enthaltend
- eine oder mehrere kosmetisch oder pharmazeutisch unbedenkliche wasserlösliche UV-Filtersubstanzen
- eine oder mehrere Substanzen, gewählt aus der Gruppe der üblichen kosmetischen oder dermatologischen Wirk- , Hilfs- und/oder Zusatzstoffe
  in einem üblichen kosmetischen oder pharmzeutischen Träger.

Als eine weitere Verkörperung der vorliegenden Erfindung wird die Verwendung einer Zusammensetzung, bestehend aus

- einem oder mehreren kosmetisch oder pharmazeutisch unbedenklichen hydrophoben anorganischen Pigmenten,
- einer oder mehreren kosmetisch oder pharmazeutisch unbedenklichen öllöslichen UV-Filtersubstanzen,
- einem oder mehreren Filmbildnern,
  zum Erzielen oder Steigern von Wasserfestigkeit kosmetischer oder dermatologischer Lichtschutzformulierungen, welche in Form von O/W-Emulsionen oder Hydrodispersionen vorliegen, und wobei die anorganischen Pigmente in die Ölphase der Emulsionen bzw. Hydrodispersionen eingebaut sind,
  angesehen.

Gleichermaßen vorteilhaft ist, wenn die erfindungsgemäßen Zubereitungen als O/W-Emulsionen oder als Hydrodispersionen vorliegen.

Zwar werden in den Schriften EP-OS 456 458, EP-OS 456 459 und EP-OS 456 460 Lichtschutzformulierungen auf der Basis von $TiO_2$-Pigmenten beschrieben. Wasserfeste Systeme, insbesondere emulgatorfreie, wasserfeste Systeme sind aber auf solche Weise nicht realisierbar. Auch sind pigmenthaltige Hydrodispersionen der erfindungsgemäßen Art bisher nicht bekannt gewesen.

Überraschend und nicht vorhersehbar war, daß bei Befolgen der hiermit offenbarten Lehre zum technischen Handeln in jeglicher Hinsicht überaus befriedigende Präparate erhältlich sind.

Liegen die erfindungsgemäßen Zubereitungen als Hydrodispersionen vor, wäre vielmehr zu erwarten gewesen, daß sich die Mikropigmentpartikel, mangels eines Emulgators, zu Agglomeraten zusammenballen würden. Ferner war überraschend, daß die Klebrigkeit der Hydrodispersionen bei Befolgung der hiermit offenbarten Lehre zum technischen Handeln drastisch vermindert werden kann.

Weiterhin war überraschend, daß, und dies gilt für O/W-Emulsionen wie auch für Hydrodispersionen, bei Befolgen der hiermit offenbarten Lehre wasserfeste Lichtschutzformulierungen mit hervorragenden Schutzeigenschaften erreichbar sind.

Zwar beschreiben R.Oteri, St.Johnson und S.Dastis in "A New Waterproofing Agent for Sunscreen Products" , Cosmetics & Toiletries 102, März 1987, S.107 - 109 einige der erfindungsgemäß vorteilhaften Filmbildner, geben jedoch keinen Hinweis darauf, daß die eventuell auf solche Weise erreichbare Wasserfestigkeit durch den Zusatz von hydrophoben anorganischen Pigmenten noch gesteigert werden kann.

Schließlich war überraschend, daß der Einsatz von anorganischen Pigmenten in den erfindungsgemäßen Formulierungen nicht zu starker Hauttrockenheit führt, sondern im Gegenteil ein anhaltendes, äußerst angenehmes Hautgefühl verursacht.

Es wird vermutet, daß die erfindungsgemäßen öllöslichen UV-Filtersubstanzen an die Oberfläche der hydrophoben anorganischen Pigmente adsorbiert werden.

Zwar beschreiben M.Schmidt und S.G.Steinemann in "XPS studies of amino acids adsorbed on titanium dioxide surfaces", Fresenius' Journal of Analytical Chemistry (1991) 341, S.412 - 415, daß gewisse Aminosäuren leicht von der Oberfläche von Titandioxidpartikeln adsorbiert werden. Ein Hinweis auf die vorliegende Erfindung und ihre vorteilhaften Eigenschaften liefert diese Arbeit jedoch keineswegs. Zudem ist das $TiO_2$, welches a.a.O. zitiert wird, nicht hydrophob.

Erfindungsgemäße kosmetische und dermatologische Zubereitungen enthalten bevorzugt anorganische Pigmente auf Basis von Metalloxiden und/oder anderen in Wasser schwerlöslichen oder unlöslichen Metallverbindungen, insbesondere der Oxide des Titans ($TiO_2$), Zinks (ZnO), Eisens (z.B. $Fe_2O_3$), Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Voraussetzung für die Verwendbarkeit anorganischer Pigmente für die erfindungsgemäßen Zwecke ist natürlich die kosmetische bzw. dermatologische Unbedenklichkeit der zugrundeliegenden Substanzen.

Im wesentlichen unerheblich für die vorliegende Erfindung ist dabei, in welchen Modifikationen solche Metalloxide vorliegen. $TiO_2$ beispielsweise kommt in der Natur in drei Hauptmodifikationen (Rutil, Anatas und Brookit) vor, welche grundsätzlich alle gleichermaßen geeignet sind. Änliches gilt für die Modifikationen der Eisenoxide usw.

Vorteilhaft ist, den Partikeldurchmesser der verwendeten Pigmente kleiner als 100 nm zu wählen.

Erfindungsgemäß liegen die anorganischen Pigmente in hydrophober Form vor, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Reaktion gemäß

$$n\ TiO_2 + m\ (RO)_3Si\text{-}R' \rightarrow TiO_2\ (oberfl.)$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handelsbezeichnungen T 805 von der Firma DEGUSSA oder M 262 von der Firma KEMIRA oder MT 100 T von der Firma TAYCA erhältlich.

Als Filmbildner werden Stoffe angesehen, die nach Lösung in Wasser oder organischen Lösemitteln auf die Haut aufgetragen werden und nach Abdunsten des Lösemittels auf der Haut einen mehr oder weniger gut zusammenhängenden Film hinterlassen.

Insbesondere vorteilhaft im Sinne der vorliegenden Erfindung sind öllösliche bzw. hydrophobe Filmbildner wie hydrophobisierte Polyvinylpyrrolidonderivate, beispielsweise Copolymere der Polyvinylpyrrolidone mit Hexadecen bzw. Eicosen.

Aber auch die Wasserfestigkeit von Zubereitungen, enthaltend wasserlösliche bzw. hydrophile Filmbildner kann erfindungsgemäß erheblich gesteigert werden. Erfindungsgemäß vorteilhaft verwendet werden können daher auch solche Filmbildner, insbesondere gewählt aus der Gruppe der Verdicker bzw. Gelbildner, bevorzugt gewählt aus der Gruppe der Polyacrylate, insbesondere der Carbopole, von diesen bevorzugt die Typen 980, 981, 1382, 2984, 5984, jeweils einzeln oder in Kombination, aber auch der Polysaccharide bzw. deren Derivate, z.B Hyaluronsäure, Xanthangummi, Hydroxyalkylcellulose, beispielsweise Hydroxypropylmethylcellulose, Polyvinylpyrrolidone.

Vorteilhaft können öllösliche bzw. hydrophobe Filmbildner zusammen mit wasserlöslichen bzw. hydrophilen Filmbildnern verwendet werden.

Als besonders erstaunliche Eigenschaft der vorliegenden Erfindung kann angesehen werden, daß erfindungsgemäß sogar relativ gut wasserlösliche oder wenigstens hydrophile Filmbildner wie Carbomer-Derivate bzw. Cellulosederivate, beispielsweise Hydroxypropylmethylcellulose, verwendet werden können und trotzdem hohe Wasserfestigkeit der erfindungsgemäßen Zubereitungen gewährleistet ist.

Aber auch bei wasserunlöslichen bzw. hydrophoben Filmbildnern ist durch die Anwendung der erfindungsgemäßen Lehre eine deutliche Steigerung der Wasserfestigkeit zu erzielen.

Als weitere vorteilhafte Verkörperung der vorliegenden Erfindung wird daher angesehen die Verwendung von Zusammensetzungen, enthaltend

- ein oder mehrere kosmetisch oder pharmazeutisch unbedenkliche hydrophobe anorganische Pigmente, wobei diese Pigmente in die Ölphase der Emulsionen bzw. Hydrodispersionen eingebaut sind,

  - wobei die Hydrophobierung nicht auf einer Aluminiumstearatbehandlung beruht

- eine oder mehrere kosmetisch oder pharmazeutisch unbedenkliche öllösliche UV-Filtersubstanzen,
  zum Erzielen oder Steigern der Wasserfestigkeit kosmetischer oder dermatologischer Lichtschutzzubereitungen, welche in Form von O/W-Emulsionen oder Hydrodispersionen vorliegen und einen oder mehrere öllösliche bzw. hydrophobe und/oder wasserlösliche bzw. hydrophile Filmbildner enthalten.

Erfindungsgemäße wasserfeste Lichtschutzzubereitungen sind vorteilhaft durch

- einen Gehalt von 0,1 bis 15 Gew.-%, insbesondere 0,5 bis 8,0 Gew.-%, an öllöslichen UV-Filtersubstanzen und/oder
- einen Gehalt von 0,1 bis 10 Gew.-%, insbesondere 0,5 bis 5,0 Gew.-% an hydrophoben anorganischen Pigmenten und/oder
- einen Gehalt von 0,1 bis 10 Gew.-%, insbesondere 0,5 bis 3,0 Gew.-% an Filmbildnern
gekennzeichnet, jeweils bezogen auf das Gesamtgewicht der Zusammensetzung.

Die Herstellung erfindungsgemäßer O/W-Emulsionen bzw. Hydrodispersionen geschieht nach den üblichen, dem Fachmanne geläufigen Regeln.

Es ist möglich und vorteilhaft, das oder die hydrophoben anorganischen Pigmente und die öllöslichen UV-Filtersubstanz bzw. -substanzen zu jedem beliebigen Zeitpunkte der Emulsionsherstellung dem Emulsionsgemisch zuzugeben. Dabei können Pigment oder Pigmente und öllösliche UV-Filtersubstanz bzw. -substanzen sowohl getrennt als auch bereits miteinander vereinigt dem Emulsionsgemisch zugegeben werden.

Es wird bevorzugt, das hydrophobe anorganische Pigment und/oder die öllöslichen UV-Filtersubstanz bzw. -substanzen der Ölphase einzuverleiben und die Ölphase hernach mit der Wasserphase zu vereinigen.

Ferner wird bevorzugt, wenn der oder die Filmbildner der jeweiligen Phase einverleibt werden, zu der sie eine besondere Affinität besitzen. Wasserlösliche bzw. hydrophile Filmbildner werden also bevorzugt der Wasserphase, öllösliche bzw. lipophile Filmbildner bevorzugt der Ölphase zugegeben.

Besonders vorteilhafte Zubereitungen werden ferner erhalten, wenn die erfindungsgemäßen Wirkstoffe mit Antioxidantien kombiniert werden. Erfindungsgemäß enthalten die O/W-Emulsionen bzw. Hydrodispersionen vorteilhaft ein oder mehrere Antioxidantien. Als günstige, aber dennoch fakultativ zu verwendende Antioxidantien alle für kosmetische und/oder dermatologische Anwendungen geeigneten oder gebräuchlichen Antioxidantien verwendet werden.

Besonders vorteilhaft werden die Antioxidantien gewählt aus der Gruppe bestehend aus Ascorbinsäure (Vitamin C), Ascorbinsäurederivaten, den verschiedenen Tocopherolen (Vitamin E) und Tocopherylestern bzw. anderen Tocopherolderivaten, Folsäure (früher als Vitamin $B_c$, $B_9$, oder M bezeichnet, heute der Vitamin $B_2$-Gruppe zugeordnet), Phytinsäure (Inosithexaphosphorsäure, auch Fytinsäure), den verschiedenen Ubichinonen (Mitochinone, Coenzyme Q), Gallenextrakt, cis- und/oder trans-Urocaninsäure (4-Imidazolylacrylsäure), Carnosin (N-β-Alanyl-L-histidin, Ignotin), Histidin, Flavonen oder Flavonoiden, Cystin (3,3'-Dithiobis(2-aminopropionsäure), Cystein (2-Amino-3-mercaptopropionsäure) und dessen Derivaten (z.B. N-Acetylcystein), die verschiedenen Carotine (insbesondere β-Carotin und Lycopin (Psi-Carotin)), Tyrosin (2-Amino-3-(4-Hydroxyphenyl)-propionsäure),_-Liponsäure (1,2-Dithiolan-3-pentansäure), Glutathion (gamma-L-Glutamyl-L-cysteinglycin) und Glutathionester, Furalglucitol (Sorbitylfurfural), Mannit sowie Zinkoxid und Zinksalze (z.B.$ZnSO_4$).

Es war nicht vorauszusehen gewesen, daß die erfindungsgemäße Kombinationen mit Antioxidantien zu hautverträglichen Produkten führen bzw. deren Verträglichkeit steigern und bei gesunder Haut nicht in die hauteigene Mikroflora eingreifen.

Vorteilhaft können die erfindinngsgemäßen öllöslichen UV-Filtersubstanzen gewählt werden aus der Gruppe der Substanzen, die UV-Strahlung vorwiegend im UVB-Bereich absorbieren, wobei die Gesamtmenge der Filtersubstanzen z.B. 0,1 Gew.-% bis 15 Gew.-%, vorzugsweise 0,5 bis 10 Gew.-%, insbesondere 0,5 bis 8,0 Gew.-% beträgt, bezogen auf das Gesamtgewicht der Zubereitung.

Vorteilhafte öllösliche UVB-Filter sind z.B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino(p-carbo-2'-ethyl-1'-hexyloxy)-1,3,5-triazin.

Es ist gegebenenfalls vorteilhaft, den erfindungsgemäßen Zubereitungen zusätzlich auch wasserlösliche UV-Filtersubstanzen einzuverleiben. Vorteilhafte wasserlösliche UVB-Filter sind z.B.:

- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Ganz besonders vorteilhaft werden die erfindungsgemäßen öllöslichen UV-Filtersubstanzen gewählt aus der Gruppe der öllöslichen Substanzen, die UV-Strahlung vorwiegend im UVA-Bereich absorbieren. Öllösliche UVA-Filter, die erfindungsgemäß vorteilhaft verwendet werden können, sind beispielsweise Derivate des Dibenzoylmethans, insbesondere 1-(4'-tert.Butylphenyl)-3-(4'-methoxyphenyl)propan-1,3-dion und um 1-Phenyl-3-(4'-isopropylphenyl)propan-1,3-dion.

Die Gesamtmenge der UVA-Filtersubstanzen kann vorteilhaft 0,1 Gew.-% bis 15 Gew.-%, vorzugsweise 0,5 bis 10 Gew.-%, insbesondere 0,5 bis 8,0 Gew.-% betragen, bezogen auf das Gesamtgewicht der Zubereitung.

Emulsionen gemäß der Erfindung z.B. in Form einer Sonnenschutzcreme oder einer Sonnenschutzmilch sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Vorteilhaft sind erfindungsgemäß solche kosmetische und dermatologische Zubereitungen, die in der Form eines Sonnenschutzmittels, eines Pré-Soleil- oder Après-Soleil-Produktes vorliegen. Vorteilhaft enthalten diese zusätzlich mindestens eine UVA-Filtersubstanz und/oder mindestens eine UVB-Filtersubstanz.

Ferner sind erfindungsgemäß solche kosmetische und dermatologische Zubereitungen besonders vorteilhaft, die in der Form eines Sonnenschutzmittels, eines Pré-Soleil- oder Après-Soleil-Produktes vorliegen und zusätzlich zu dem oder den UVA-Filtern und/oder dem oder den UVB-Filtern ein oder mehrere Antioxidantien enthalten.

Die Gesamtmenge der Antioxidantien kann vorteilhaft 0,1 Gew.-% bis 30 Gew.-%, vorzugsweise 0,5 bis 10 Gew.-%, insbesondere 1 bis 6 Gew.-% betragen, bezogen auf das Gesamtgewicht der Zubereitung.

Die erfindungsgemäßen kosmetischen Zubereitungen können gewünschtenfalls ferner kosmetische Hilfsstoffe enthalten, wie sie üblicherweise in solchen Zubereitungen verwendet werden, z.B. Konservierungsmittel, Bakterizide, desodorierend wirkende Substanzen, Antitranspirantien, Insektenrepellentien, Vitamine, Mittel zum Verhindern des Schäumens, Farbstoffe, Pigmente mit färbender Wirkung, Verdickungsmittel, weichmachende Substanzen, anfeuchtende und/oder feuchthaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Schließlich können als Wirk- bzw. Zusatzstoffe mit besonders vorteilhaften Eigenschaften Aminosäuren gewählt werden, diese bevorzugt aus der Gruppe Glycin, Alanin, Valin, Leucin, Isoleucin, Phenylalanin, Tyrosin, Prolin, Hydroxyprolin, Serin, Threonin, Cystein, Methionin, Tryptophan, Arginin. Besonders bevorzugt von diesen Aminosäuren sind Arginin und, ganz besonders bevorzugt, Glycin.

Im den folgenden Beispielen werden vorteilhafte Verkörperungen der vorliegenden Erfindung aufgeführt.

**Beispiel 1**

| Sonnencrème, O/W, Lichtschutzfaktor 20 | |
|---|---|
| | Gew.-% |
| Cyclomethicon | 3,00 |
| Glycerylstearat + PEG-30 Stearat | 2,00 |
| Wollwachsalkohol | 0,10 |
| Glycerylstearat | 3,00 |
| Isopropylpalmitat | 2,00 |
| Octyldodecanol | 1,00 |
| $C_{12-15}$-Alkylbenzoat | 2,00 |

(fortgesetzt)

| Sonnencrème, O/W, Lichtschutzfaktor 20 | |
| --- | --- |
| | Gew.-% |
| Glycerin | 3,00 |
| Cetylalkohol | 3,00 |
| Myristylmyristat | 2,00 |
| Phenylbenzimidazolsulfonsäure | 3,00 |
| Tocopherylacetat | 0,50 |
| EDTA-Lösung (20 %-ig) | 0,50 |
| NaOH (45 %-ig) | 1,15 |
| Ethylalkohol | 4,00 |
| Konservierungsmittel | q.s. |
| Parfüm | q.s. |
| HydrophobesTiO$_2$, Partikelgröße < 100 nm | 2,00 |
| PVP/Eicosen Copolymer | 3,00 |
| Octylmethoxycinnamat | 4,50 |
| Butylmethoxydibenzoylmethan | 2,00 |
| Wasser, VES | ad 100,00 |

**Beispiel 2**

| Sonnencrème, O/W Lichtschutzfaktor 8 | |
| --- | --- |
| | Gew.-% |
| Cyclomethicon | 3,00 |
| Glycerylstearat + PEG-30 Stearat | 2,00 |
| Glycerylstearat | 3,00 |
| Isopropylpalmitat | 2,00 |
| Octyldodecanol | 3,00 |
| Glycerin | 3,00 |
| Cetylalkohol | 3,00 |
| Tocopherylacetat | 0,50 |
| EDTA-Lösung (20 %-ig) | 0,50 |
| NaOH | q.s. |
| Ethylalkohol | 1,50 |
| Konservierungsmittel | q.s. |
| Parfüm | q.s. |
| HydrophobesTiO$_2$, Partikelgröße < 100 nm | 1,00 |
| Octylmethoxycinnamat | 5,00 |
| Butylmethoxydibenzoylmethan | 0,50 |

(fortgesetzt)

| Sonnencrème, O/W Lichtschutzfaktor 8 | |
| --- | --- |
| | Gew.-% |
| PVP/Hexadecen Copolymer | 1,50 |
| Wasser, VES | ad 100,00 |

**Beispiel 3**

| Sonnenmilch, O/W | |
| --- | --- |
| | Gew.-% |
| Trilaureth-4 Phosphat | 0,75 |
| Triceteareth-4 Phosphat | 1,00 |
| Glycerylstearat + PEG-100 Stearat | 1,00 |
| Glycerylstearat + Ceteareth-20 | 0,80 |
| Glyceryllanolat | 0,50 |
| Isopropylpalmitat | 3,00 |
| Glycerin-Caprylat/Caproat ("Caprylic/Capric Triglyceride") | 5,00 |
| Cetylalkohol | 1,00 |
| Phenylbenzimidazolsulfonsäure | 4,00 |
| Butylhydroxytoluol | 0,06 |
| EDTA-Lösung (20 %-ig) | 0,50 |
| NaOH | q.s. |
| Konservierungsmittel | q.s. |
| Hydrophobes $TiO_2$, Partikelgröße < 100 nm | 3,00 |
| Octylmethoxycinnamat | 6,00 |
| Butylmethoxydibenzoylmethan | 0,50 |
| Methylbenzylidencampher | 3,00 |
| PVP/Eicosen Copolymer | 1,00 |
| Wasser, VES | ad 100,00 |

**Beispiel 4**

| Sonnenmilch, O/W | |
| --- | --- |
| | Gew.-% |
| Cyclomethicon | 2,00 |
| Cetearylalkohol + PEG-40-hydriertes Rizinusöl + Natriumcetearylsulfat | 2,50 |
| Glyceryllanolat | 1,00 |
| Glycerin-Caprylat/Caproat ("Caprylic/Capric Triglyceride") | 2,00 |
| Octylstearat | 3,00 |

(fortgesetzt)

| Sonnenmilch, O/W | |
|---|---|
| | Gew.-% |
| Rizinusöl | 4,00 |
| Glycerin | 3,00 |
| Butylhydroxytoluol | 0,03 |
| EDTA-Lösung (20 %-ig) | 0,50 |
| NaOH | q.s. |
| Konservierungsmittel | q.s. |
| Parfüm | q.s. |
| HydrophobesTiO$_2$, Partikelgröße < 100 nm | 1,50 |
| Carbomer 981 | 0,23 |
| Octylmethoxycinnamat | 4,00 |
| Butylmethoxydibenzoylmethan | 2,00 |
| Methylbenzylidencampher | 3,50 |
| Hydroxypropylmethylcellulose | 0,30 |
| PVP/Hexadecen Copolymer | 1,50 |
| Wasser, VES | ad 100,00 |

**Beispiel 5**

| Sonnenmilch, O/W | |
|---|---|
| | Gew.-% |
| Stearinsäure | 2,00 |
| Cetylpalmitat | 1,00 |
| Glycerin-Caprylat/Caproat ("Caprylic/Capric Triglyceride") | 2,00 |
| Sorbitanmonooleat | 1,00 |
| Sorbitanmonostearat | 1,50 |
| Paraffinöl, DAB 9 | 1,29 |
| Cetearylalkohol | 0,80 |
| Glycerin | 3,00 |
| Butylhydroxytoluol | 0,06 |
| Simethicon | 0,20 |
| Polyethylenglycol-150 | 3,00 |
| Triethanolamin | 0,85 |
| Ethylalkohol | 3,00 |
| Konservierungsmittel | q.s. |
| Parfüm | q.s. |
| HydrophobesTiO$_2$, Partikelgröße < 100 nm | 2,00 |

(fortgesetzt)

| Sonnenmilch, O/W | |
| --- | --- |
| | Gew.-% |
| Octylmethoxycinnamat | 5,00 |
| Methylbenzylidencampher | 1,00 |
| Carbomer 981 | 0,10 |
| PVP/Hexadecen Copolymer | 2,00 |
| Wasser, VES | ad 100,00 |

**Beispiel 6**

| Sonnenhydrodispersionsgel, Lichtschutzfaktor 12 | |
| --- | --- |
| | Gew.-% |
| Phenyltrimethicon | 4,50 |
| Ethanol | 9,00 |
| Glycerin | 4,50 |
| EDTA-Lösung (14 %-ig) | 0,75 |
| Trisaminopromethamin | 2,01 |
| Parfüm, Konservierungsmittel, Farbstoffe | q.s. |
| Hydrophobes $TiO_2$, Partikelgröße < 100 nm | 4,50 |
| Octylmethoxycinnamat | 7,50 |
| Parsol®1789 | 3,00 |
| Hydroxypropylmethylcellulose | 0,30 |
| Carbomer 981 | 1,50 |
| PVP/Hexadecen Copolymer | 1,00 |
| Wasser, VES | ad 100,00 |

**Beispiel 7**

| Sonnenhydrodispersionsgel, Lichtschutzfaktor 12 | |
| --- | --- |
| | Gew.-% |
| Octyldodecanol | 4,50 |
| Ethanol | 9,00 |
| Butylenglycol | 4,50 |
| EDTA-Lösung (14 %-ig) | 0,75 |
| Trisaminopromethamin | 2,01 |
| Parfüm, Konservierungsmittel, Farbstoffe | q.s. |
| Hydrophobes $TiO_2$, Partikelgröße < 100 nm | 4,50 |

(fortgesetzt)

| Sonnenhydrodispersionsgel, Lichtschutzfaktor 12 | |
|---|---|
| | Gew.-% |
| Octylmethoxycinnamat | 7,50 |
| Parsol®1789 | 3,00 |
| Carbomer 981 | 1,50 |
| Hydroxypropylmethylcellulose | 0,30 |
| PVP/Eicosen Copolymer | 1,50 |
| Wasser, VES | ad 100,00 |

**Beispiel 8**

| Sonnenhydrodispersionsgel, Lichtschutzfaktor 12 | |
|---|---|
| | Gew.-% |
| Ricinusöl | 4,50 |
| Ethanol | 9,00 |
| Butylenglycol | 4,50 |
| EDTA-Lösung (14 %-ig) | 0,75 |
| Trisaminopromethamin | 2,01 |
| Parfüm, Konservierungsmittel, Farbstoffe | q.s. |
| Hydrophobes $TiO_2$, Partikelgröße < 100 nm | 4,50 |
| Octylmethoxycinnamat | 7,50 |
| Parsol®1789 | 3,00 |
| Carbomer 981 | 1,50 |
| Hydroxypropylmethylcellulose | 0,30 |
| Wasser, VES | ad 100,00 |

**Patentansprüche**

1. Wasserfeste kosmetische oder dermatologische Lichtschutzformulierungen in Form von O/W-Emulsionen oder Hydrodispersionen, enthaltend

   - ein oder mehrere kosmetisch oder pharmazeutisch unbedenkliche hydrophobe anorganische Pigmente, wobei diese Pigmente in die Ölphase der Emulsionen bzw. Hydrodispersionen eingebaut sind,

     - wobei, falls Titandioxid als hydrophobes anorganisches Pigment eingesetzt wird, die Hydrophobierung des Titandioxids nicht auf einer Aluminiumstearatbehandlung beruht

   - eine oder mehrere kosmetisch oder pharmazeutisch unbedenkliche öllösliche UV-Filtersubstanzen,
   - einen oder mehrere Filmbildner
     ferner gegebenenfalls enthaltend
   - eine oder mehrere kosmetisch oder pharmazeutisch unbedenkliche wasserlösliche UV-Filtersubstanzen
   - eine oder mehrere Substanzen, gewählt aus der Gruppe der üblichen kosmetischen oder dermatologischen Wirk-, Hilfs- und/oder Zusatzstoffe
     in einem üblichen kosmetischen oder pharmzeutischen Träger.

2. Verwendung einer Zusammensetzung, bestehend aus

- einem oder mehreren kosmetisch oder pharmazeutisch unbedenklichen hydrophoben anorganischen Pigmenten,
- einer oder mehreren kosmetisch oder pharmazeutisch unbedenklichen öllöslichen UV-Filtersubstanzen,
- einem oder mehreren Filmbildnern,
  zum Erzielen oder Steigern von Wasserfestigkeit kosmetischer oder dermatologischer Lichtschutzformulierungen, welche in Form von O/W-Emulsionen oder Hydrodispersionen vorliegen, und wobei die anorganischen Pigmente in die Ölphase der Emulsionen bzw. Hydrodispersionen eingebaut sind.

3. Verwendung von Zusammensetzungen, enthaltend

- ein oder mehrere kosmetisch oder pharmazeutisch unbedenkliche hydrophobe anorganische Pigmente,

  - wobei die anorganischen Pigmente in die Ölphase der Emulsionen bzw. Hydrodispersionen eingebaut sind

- eine oder mehrere kosmetisch oder pharmazeutisch unbedenkliche öllösliche UV-Filtersubstanzen,
  zum Erzielen oder Steigern der Wasserfestigkeit kosmetischer oder dermatologischer Lichtschutzzubereitungen, welche in Form von O/W-Emulsionen oder Hydrodispersionen vorliegen und einen oder mehrere öllösliche bzw. hydrophobe und/oder wasserlösliche bzw. hydrophile Filmbildner enthalten.

**Claims**

1. Water-resistant cosmetic or dermatological light protection formulations in the form of O/W emulsions or hydrodispersions, comprising

- one or more cosmetically or pharmaceutically acceptable hydrophobic inorganic pigments, these pigments being incorporated into the oily phase of the emulsions or hydrodispersions,

  - wherein, if titanium dioxide is used as the hydrophobic inorganic pigment, the hydrophobization of the titanium dioxide is not based on an aluminium stearate treatment,

- one or more cosmetically or pharmaceutically acceptable oil-soluble UV filter substances,
- one or more film-forming agents
  and furthermore, comprising, if appropriate,
- one or more cosmetically or pharmaceutically acceptable water-soluble UV filter substances
- one or more substances chosen from the group consisting of the customary cosmetic or dermatological active compounds, auxiliaries and/or additives
  in a customary cosmetic or pharmaceutical carrier.

2. Use of a composition comprising

- one or more cosmetically or pharmaceutically acceptable hydrophobic inorganic pigments,
- one or more cosmetically or pharmaceutically acceptable oil-soluble UV filter substances,
- one or more film-forming agents
  for achieving or increasing the water resistance of cosmetic or dermatological light protection formulations which are in the form of O/W emulsions or hydrodispersions, the inorganic pigments being incorporated into the oily phase of the emulsions or hydrodispersions.

3. Use of compositions comprising

- one or more cosmetically or pharmaceutically acceptable hydrophobic inorganic pigments,

  - wherein the inorganic pigments are incorporated into the oily phase of the emulsions or hydrodispersions,

- one or more cosmetically or pharmaceutically acceptable oil-soluble UV filter substances,
  for achieving or increasing the water resistance of cosmetic or dermatological light protection formulations which are in the form of O/W emulsions or hydrodispersions and comprise one or more oil-soluble or hydrophobic and/or water-soluble or hydrophilic film-forming agents.

**Revendications**

1. Formulations de protection solaire, cosmétiques ou dermatologiques, résistantes à l'eau, sous la forme d'émulsions H/E [huile/eau] ou d'hydrodispersions, contenant

   - un ou plusieurs pigments inorganiques, hydrophobes, inoffensifs du point de vue cosmétique ou pharmaceutique, ces pigments étant incorporés dans la phase huileuse des émulsions ou des hydrodispersions,

   - l'hydrophobisation du dioxyde de titane, en cas d'utilisation du dioxyde de titane en tant que pigment inorganique hydrophobe, n'étant pas basée sur un traitement au stéarate d'aluminium,

   - une ou plusieurs substances-filtre UV, liposolubles, inoffensives du point de vue cosmétique ou pharmaceutique,

   - un ou plusieurs agents filmogènes,
     contenant de surcroît, le cas échéant,

   - une ou plusieurs substances-filtre UV hydrosolubles, inoffensives du point de vue cosmétique ou pharmaceutique,

   - une ou plusieurs substances, choisies parmi le groupe des agents actifs, des auxiliaires et/ou des adjuvants cosmétiques ou pharmaceutiques usuels,
     dans un excipient cosmétique ou pharmaceutique usuel.

2. Utilisation d'une composition, se composant

   - d'un ou de plusieurs pigments inorganiques hydrophobes, inoffensifs du point de vue cosmétique ou pharmaceutique,

   - d'une ou de plusieurs substances liposolubles, inoffensives du point de vue cosmétique ou pharmaceutique,

   - d'un ou plusieurs agents filmogènes,
     en vue de l'obtention ou de l'accroissement de la résistance à l'eau de formulations cosmétiques ou dermatologiques de protection solaire, qui sont présentes sous la forme d'émulsions H/E ou d'hydrodispersions, et les pigments inorganiques étant incorporés dans la phase huileuse des émulsions ou des hydrodispersions.

3. Utilisation de compositions, contenant

   - un ou plusieurs pigments inorganiques hydrophobes inoffensifs du point de vue cosmétique ou pharmaceutique,

   - ces pigments étant incorporés dans la phase huileuse des émulsions ou des hydrodispersions,

   - une ou plusieurs substances liposolubles, hydrophobes inoffensives du point de vue cosmétique ou pharmaceutique,
     en vue de l'obtention ou de l'accroissement de la résistance à l'eau de formulations cosmétiques ou dermatologiques de protection solaire, qui sont présentes sous la forme d'émulsions H/E ou d'hydrodispersions, et qui contiennent un ou plusieurs agents filmogènes liposolubles ou hydrophobes, et/ou hydrosolubles ou hydrophiles.